# EUROPEAN PATENT APPLICATION

(11) **EP 4 368 168 A1**
(43) Date of publication of application: **15.05.2024**
(21) Application number: 22206924.7
(22) Date of filing: 11.11.2022
(51) Int. Cl.: A61K 8/34, A61K 8/365, A61Q 19/06

(54) **COMPOSITION FOR ANTI-CELLULITE TREATMENTS**

(71) Applicant: Voityla, Ringoldas, 08400 Vilnius (LT)
(72) Inventor: Voityla, Ringoldas, 08400 Vilnius (LT)
(74) Representative: Zaboliene, Reda

(57) **Abstract**

The present invention provides a method for the preparation of composition used for the treatment of cellulite, especially cellulite lumps. The present invention relates to an anticellulite composition that consist of ethyl alcohol and citric acid. The composition is high quality solution, is very effective and easy to use. The duration of the treatment depends on the size of cellulite lumps, at least from 5 months to 2 years. Prepared composition is colourless solution which should be applied 2-3 times per day on the problematic places of your body. The composition is applied on the dry skin desired area, and it is remained to dry for 15-30 seconds.

## Description

### TECHNICAL FIELD

The present invention concerns a topical composition for the treatment of cellulite. More particular, the invention refers to a solution, suitable to be administered cutaneously, by applying directly on skin or by spray.

### BACKGROUND

Cellulite is commonly understood to mean the accumulation of adipose tissue in certain body areas, to form adipose fat nodules. Between 85% and 98% of women after puberty present some degree of cellulite, which is conditioned by genetics and ethnic origin, and is more frequent, for example, in Mediterranean women. In order to prevent its appearance, good nutrition and habitual exercise are recommended, supplemented by topically-applied cosmetic products.

Cellulite is caused by degeneration of the microcirculation of adipose tissue with consequent alteration of its most important metabolic functions. Cellulite is a common and harmless skin condition that causes dimpled or lumpy textured skin. With cellulite, the lumps are caused by the collagen strands tightening, pulling the skin down, and the bumps or nodules are the result of fat cells pushing against the skin. It is worth noting that cellulite isn't just excess fat, which is why very thin or toned women still see cellulite lumps and bumps. Rather, cellulite is the result of fat cells pushing up against the skin, constricted by tight, short, fibrous bands in the dermis.

Currently, there are hundreds of products in the market designed to prevent or reduce cellulite, with various degrees of complexity in their composition and more or less costly. These compositions present certain problems: on the one hand, their formulations include chemical products that cause such compounds to exhibit secondary effects in the short or the long term; on the other hand, their results do not reach a desirable level, for which reason none of them combines the four basic, essential actions that are recognised as desirable in the treatment of cellulite, i.e. lipolysis, anti-oedemic properties, circulation activation and regulation of capillary permeability.

KR20080041079 discloses A cosmetic composition for anti-cellulite contains caffeine, horsetail and holly extracts. The horsetail and holly extracts are isolated from leaves of the horsetail and holly, respectively with one solvent selected from methanol, ethanol, isopropanol, butanol, acetone and ethylmethylketone and mixture with water. The content of horsetail and holly extract is 0.1-20 weight% based on total weight. As a result of examining the most important cellulite decomposition effect in the present invention, it was found that 1.00% of the extract mixture was most suitable as an anti-cellulite cream. CN201510620447 discloses the invention which relates to a relaxing and firming eye mask and a preparation method thereof. The composition among ethanol and citric acid comprises many other parts. The eye mask can improve circulation in skin around eyes and accelerate skin metabolism, thereby effectively relieving eye pouches, fading black eyes and reducing fine lines, it can also eliminate cellulite.

The goal of this invention is to prepare very simple and effective preparation for use in the treatments of cellulite, especially such as to avoid the cellulite lumps and unsightly look of the skin in the area with cellulite in women and men.

These and other results are obtained according to the present invention by providing pharmaceutical composition, suitable to be applied topically on the skin, the action of which is based on the combination of two active ingredients never used before for this kind of treatment.

Experimental data shows that invented composition is very effective in the treatment of cellulite, especially cellulite lumps. The composition does not contain toxic ingredients, and toxic reagents are not used in the preparation of it. The composition is high quality solution, which is very effective and easy to use.

### BRIEF DESCRIPTION OF THE INVENTION

The present invention provides a method for the preparation of composition used for the treatment of cellulite, especially cellulite lumps. It would be much better to dispose of a pharmaceutical composition, for topical use, that can be easily applied to the body in a "do-it-yourself' way but allowing to achieve appreciable results.

The present invention relates to an anti-cellulite composition that consist of ethyl alcohol, which has formula C₂H₅OH, and citric acid, which has a formula C₆H₈O₇, wherein citric acid can be changed by natural lemon juice.

The composition is intended for use in the treatment of cellulite, wherein natural lemon juice or citric acid is added to the pure spirit until the concentration of the solution reaches from 92,5 to 92.7%.

Prepared composition is colourless solution which is applied 2-3 times per day on the problematic places of your body. The composition is applied on the dry skin, and it is remained to dry for 15-30 seconds. The duration of the treatment depends on the size of cellulite lumps, at least from 5 months to 2 years.

The composition is presented in the form of a solution or spray. The cosmetic composition is applied on the dry desired skin area remaining the skin to dry for 15-30 seconds.

The composition is used for the treatment of condition selected form cellulite, lipogenesis, lipolysis, lipid quantity contained in subcutaneous fat tissue.

### DETAILED DESCRIPTION OF THE INVENTION

An aim of the present invention is therefore that of providing a pharmaceutical composition allowing to overcome the limits of the anti-cellulite products according to the prior art and to obtain the results described previously. A further aim of the invention is that said formulation can be realised with substantially limited costs, as far as both the costs of the used compounds and the costs of production is concerned. The present invention relates to an anti-cellulite composition which consist of ethyl alcohol and citric acid. In the present invention, as the citric acid the natural lemon juice can be used.

Both ingredients are mixed to the solution concentration from 90 to 95%. The preferred concentration is from 92,5 to 92.7%.

The composition of the invention may be presented in the form of solution or spray.

The present invention relates to an anti-cellulite composition that consist of two ingredients. The first ingredient is ethyl alcohol (C₂H₅OH), the second ingredient is selected from citric acid (C₆H₈O₇). Both ingredients are mixed to the solution concentration from 90 to 95%. The preferred concentration is from 92,5 to 92.7%. Prepared solution is filled into bottles. Another aspect of the invention relates to a process for obtaining the composition described above, which comprises the step of mixing of the ethyl alcohol with natural lemon juice. Since one of the options to produce the composition is the use of natural lemon juice, an aerometer is used to obtain the composition. Natural lemon juice is added to the ethyl alcohol, constantly measuring the concentration of the solution. The solution is considered ready when its concentration is from 92,5 to 92.7%.

Preferably, this process is performed at an ambient temperature.

Another aspect relates to a cosmetic composition used for the reduction and/or elimination of cellulite, especially cellulite lumps, which comprises the following steps:
a. applying the composition described above on the dry desired area,
b. remaining the skin to dry for 15-30 seconds.

Prepared composition is applied 2-3 times per day on the problematic places of your body. The composition is applied on the dry interested skin zone, and it is remained to dry for 15-30 seconds so to allow a complete absorption. The patient is not allowed to have a shower or put water after applying the composition on the skin or apply the composition on the injured skin.

The duration of the treatment depends on the size of cellulite lumps, at least from 5 months to 2 years.

Preferably, the application of step (a) is performed by means of tampon or directly with a spray.

For persons skilled in the art, other objects, advantages and characteristics of the invention will arise partly from the description and partly from the practice of the invention. The following example is provided for illustrative purposes and is not intended to limit the scope of this invention.

The particular embodiments disclosed above are illustrative only, as the present disclosure may be modified and practiced in different but equivalent manners apparent to those skilled in the art having the benefit of the teachings herein. Although individual embodiments are discussed, the disclosure covers all combinations of all those embodiments.

## Claims

1. A cosmetic composition that consist of:
ethyl alcohol which formula is C₂H₅OH and
citric acid which formula is C₆H₈O₇,
for use in the treatment of cellulite, wherein citric acid is added to the ethyl alcohol until the concentration of the solution reaches from 92,5 to 92.7%.

2. Composition for use according to claim 1, wherein citric acid can be changed by natural lemon juice.

3. Composition for use according to claim 1, wherein said solution it is presented in the form of a solution or spray.

4. The cosmetic composition for use according to any of claims 1 - 3, wherein the cellulite condition is selected form cellulite, lipogenesis, lipolysis, lipid quantity contained in subcutaneous fat tissue.

5. Use of the cosmetic composition according to any of preceding claims, which comprises the following steps:
a. applying the composition described above on the dry skin desired area,
b. remaining the skin to dry for 15-30 seconds.

6. Use of the cosmetic composition according to claim 5, where the application of step (a) is performed by applying the solution directly or with a spray.

7. Use of the cosmetic composition according to claims 5 or 6, where the composition is applied topically on the cellulite lumps.

8. Use of the cosmetic composition according to claims 5 or 6, where the composition is sprayed on cellulite I stage and II stage.

9. Use of the composition according to any of claims 5-8, which is applied 2-3 times per day on the dry skin desired area of man or women.
